# DEMANDE DE BREVET EUROPEEN

(11) **EP 1 245 226 A1**
(43) Date de publication de la demande: **02.10.2002**
(21) Numéro de dépôt: 02290686.1
(22) Date de dépôt: 19.03.2002
(51) Int. Cl.: A61K 7/50

(54) **Compositions cosmétiques détergentes contenant un tensioactif anionique dérivé d'acides amines**

(30) Priorité: 30.03.2001 FR 0104380
(71) Demandeur: L'OREAL, 75008 Paris (FR)
(72) Inventeur: Perron, Beatrice, 78350 Jouy en Josas (FR); Restle Serge, 95390 Saint Prix (FR)
(74) Mandataire: Le Blainvaux Bellegarde, Françoise

(57) **Abrégé**

L'invention concerne de nouvelles compositions cosmétiques détergentes comprenant dans un milieu cosmétiquement acceptable au moins un tensioactif anionique choisi parmi les composés N-acylés d'aminoacides polycarboxyliques mono ou poly amidifiés et leurs sels, et au moins un agent conditionneur soluble particulier.

Cette association apporte des propriétés cosmétiques (lissage, légèreté, douceur) sans phénomène de regraissage des fibres kératiniques.

Ces compositions sont utilisées notamment pour le lavage et/ou le conditionnement des matières kératiniques telles que les cheveux ou la peau.

## Description

La présente invention concerne de nouvelles compositions cosmétiques comprenant dans un milieu cosmétiquement acceptable au moins un tensioactif anionique choisi parmi les composés N-acylés d'aminoacides polycarboxyliques mono ou poly amidifiés et leurs sels et au moins un agent conditionneur soluble particulier.

Pour le nettoyage et/ou le lavage des cheveux et/ou de la peau, l'utilisation de compositions détergentes (shampooing ou gel-douche) à base essentiellement d'agents tensioactifs classiques de type notamment anionique, non ionique et/ou amphotère, mais plus particulièrement de type anionique, est courante. Ces compositions sont appliquées sur cheveux ou peau mouillés et la mousse générée par massage ou friction avec les mains permet, après rinçage à l'eau, l'élimination des diverses salissures initialement présentes sur les cheveux ou la peau.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur les matières kératiniques des dommages plus ou moins marqués liés en particulier à l'élimination progressive des lipides ou protéines contenues dans ou à la surface de ces dernières.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés siliconés, qui confèrent en effet aux cheveux lavés, secs ou mouillés, une facilité de démêlage, une douceur et un lissage accrus par rapport à ce qui peut être obtenu avec les compositions nettoyantes correspondantes qui en sont exemptes.

Toutefois, et malgré les progrès réalisés récemment dans le domaine des shampooings à base de polymères cationiques et/ou de silicone, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoquées ci-avant, de meilleures performances.

Les tensioactifs anioniques N-acylés d'aminoacides polycarboxyliques mono ou poly amidifiés et leurs sels ont déjà été préconisés dans des compositions cosmétiques détergentes. Ils ont été décrits par exemple dans la demande de brevet WO97/03171.

Les compositions de lavage des cheveux utilisant ces tensioactifs seuls ne conduisent pas à de propriétés cosmétiques satisfaisantes.

L'invention a donc pour but de proposer des compositions cosmétiques détergentes présentant des propriétés cosmétiques améliorées, en particulier le démêlage, le lissage et la douceur des cheveux.

Or, la demanderesse a maintenant trouvé que l'association de d'agents conditionneurs solubles particuliers et d'un tensioactif anionique particulier et leurs sels permettait d'atteindre ces buts.

Ces nouvelles compositions permettent de mieux déposer ces agents conditionneurs sur les matières kératiniques (notamment les cheveux) qu'une composition contenant des tensioactifs anioniques classiques tels que les sels d'acide N-cocoylglutamique.

Les compositions conformes à l'invention confèrent aux matières kératiniques notamment les cheveux, un remarquable effet traitant qui se manifeste notamment par une facilité de démêlage, ainsi qu'un apport de légèreté, de lissage, de douceur et de souplesse sans aucune sensation de toucher chargé.

L'invention a ainsi pour objet une composition cosmétique détergente, caractérisée en ce qu'elle comprend, dans un milieu cosmétiquement acceptable, au moins un tensioactif anionique choisi parmi les composés N-acylés d'aminoacides polycarboxyliques mono ou poly amidifiés et leurs sels et au moins un agent conditionneur de préférence soluble choisi parmi :
I) les polymères anioniques,
II) les polymères non ioniques,
III) les polymères cationiques,
IV) les polymères amphotères,
V) les protéines cationiques,
VI) les hydrolysats de protéines cationiques et
VII) les tensioactifs cationiques choisis parmi les sels d'ammonium quaternaires comportant au moins une fonction amide, les sels d'ammonium quaternaires de l'imidazoline, les sels de diammonium quaternaires, les sels d'ammonium quaternaire contenant au moins une fonction ester.

Un autre objet de l'invention concerne l'utilisation d'au moins un tensioactif anionique choisi parmi les composés N-acylés d'aminoacides polycarboxyliques mono ou poly amidifiés et leurs sels dans, ou pour la fabrication d'une composition cosmétique comprenant au moins un agent conditionneur de préférence soluble tel que défini ci-dessus.

Un autre objet de l'invention concerne un procédé de traitement des matières kératiniques, telles que les cheveux, caractérisé en ce qu'il consiste à appliquer sur lesdites matières des compositions cosmétiques selon l'invention.

L'invention a encore pour objet l'utilisation d'une composition selon l'invention pour améliorer le démêlage ou le lissage des cheveux , ou pour apporter du volume, de la légèreté, de la douceur, de la souplesse ou de la discipline aux cheveux.

Selon la présente invention, par matières kératiniques, on comprend les cheveux, les cils, les sourcils, la peau, les ongles, les muqueuses ou le cuir chevelu et plus particulièrement les cheveux.

Les différents objets de l'invention vont maintenant être détaillés. L'ensemble des significations et définitions des composés utilisés dans la présente invention données ci-dessous sont valables pour l'ensemble des objets de l'invention.

Les tensioactifs anioniques N-acylés d'aminoacides polycarboxyliques mono ou poly amidifiés et ses sels peuvent avoir la formule suivante (I) : 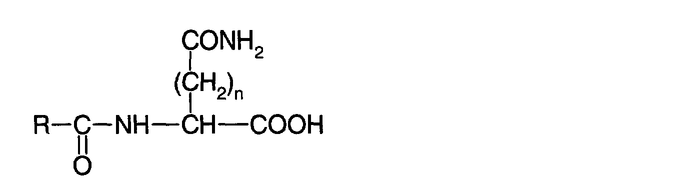 dans laquelle :
R désigne un radical hydrocarboné, saturé ou insaturé, linéaire ou ramifié comportant de 5 à 29 atomes de carbone.
R désigne de préférence un radical alkyle ou alkényle mono ou polyinsaturé comportant de 5 à 29 atomes de carbone et de préférence de 7 à 22 atomes de carbone.
n est égal à 1 ou 2.

Des composés N-acylés d'aminoacides polycarboxyliques mono ou poly amidifiés préférés selon la présente invention sont des composés de formule (I) dans laquelle R désigne plus particulièrement un radical alkyle saturé, linéaire ou ramifié comportant de 7 à 29 atomes de carbone, de préférence de 7 à 22 atomes de carbone.

Les sels des composés de formule (I) peuvent être des sels de métaux alcalins (par exemple sodium ou potassium), de métaux alcalinoterreux (par exemple calcium, magnésium), les sels d'ammoniaque, les sels d'amines comme ceux de la monoéthanolamine, la diéthanolamine, la triéthanolamine, l'amino-3 propanediol-1,2, les sels ammoniums issus des aminoacides basiques tels que la lysine, l'arginine, la sarcosine, l'ornithine, la citrulline.

Parmi les tensioactifs de formule (I), on peut citer plus particulièrement les sels de N-cocoylglutamine et notamment le sel de triéthanolamine de N-cocoylglutamine tel que le produit commercialisé sous la dénomination FOAM UP DOUCE GM par la société KYOWA HAKKO.

Selon l'invention, le ou les tensioactifs anioniques choisis parmi les composés N-acylés d'aminoacides polycarboxyliques mono ou poly amidifiés et leurs sels peuvent représenter de 1 % à 30 % en poids, de préférence de 3 % à 15 % en poids par rapport au poids total de la composition finale.

Par agents conditionneurs solubles dans l'eau, on entend des agents conditionneurs solubles dans l'eau à une concentration supérieure ou égale à 0,1% en poids dans l'eau à 25°C, c'est à dire qu'ils forment une solution macroscopiquement isotrope transparente.

Les polymères anioniques généralement utilisés sont des polymères comportant des groupements dérivés d'acide carboxylique, sulfonique ou phosphorique et ont un poids moléculaire moyen en poids allant de environ 500 à 5.000.000 déterminé par exemple par chromatographie par perméation de gel.
1 ) Les groupements carboxyliques sont apportés par des monomères mono ou diacides carboxyliques insaturés tels que ceux répondant à la formule : 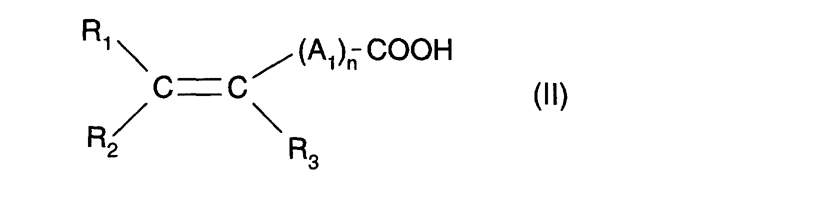 dans laquelle n est un nombre entier de 0 à 10, A₁ désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₁ désigne un atome d'hydrogène, un groupement phényle ou benzyle, R₂ désigne un atome d'hydrogène, un groupement alkyle inférieur ou carboxyle, R₃ désigne un atome d'hydrogène, un groupement alkyle inférieur, un groupement -CH₂-COOH, phényle ou benzyle ;
   Dans la formule précitée un radical alkyle inférieur désigne de préférence un groupement ayant 1 à 4 atomes de carbone et en particulier, méthyle et éthyle.
   Les polymères filmogènes anioniques à groupements carboxyliques préférés selon l'invention sont :
   A) Les homo- ou copolymères d'acide acrylique ou méthacrylique ou leurs sels et en particulier les produits vendus sous les dénominations VERSICOL E ou K par la société ALLIED COLLOID et ULTRAHOLD par la société BASF. Les copolymères d'acide acrylique et d'acrylamide vendus sous la forme de leur sel de sodium sous les dénominations RETEN 421, 423 ou 425 par la Société HERCULES, les sels de sodium des acides polyhydroxycarboxyliques.
   B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés. De tels polymères sont décrits en particulier dans le brevet français 1.222.944 et la demande allemande 2.330.956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀ par exemple de lauryle tel que celui vendu par la société ISP sous la dénomination ACRYLIDONE LM et les terpolymères acide méthacrylique/ acrylate d'éthyle/ acrylate de tertiobutyle tel que le produit vendu sous la dénomination LUVIMER 100 P par la société BASF.
   C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un ester vinylique, allylique ou méthallylique d'un acide carboxylique α- ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 vendues par la société NATIONAL STARCH.
   D) les copolymères dérivés d'acides ou d'anhydrides carboxyliques monoinsaturés en C₄-C₈ choisis parmi :
      - les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. ; De tels polymères sont décrits en particulier dans les brevets US 2.047.398, 2.723.248, 2.102.113, le brevet GB 839.805 et notamment ceux vendus sous les dénominations GANTREZ AN ou ES par la société ISP.
      - les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
         les fonctions anhydrides de ces copolymères étant éventuellement monoestérifiées ou monoamidifiées.
         Ces polymères sont par exemple décrits dans les brevets français 2.350.384 et 2.357.241 de la demanderesse.
   E) les polyacrylamides comportant des groupements carboxylates.
2) Les polymères comprenant les groupements sulfoniques sont des polymères comportant des motifs vinylsulfonique, styrène sulfonique, naphtalène sulfonique ou acrylamido alkylsulfonique.

Ces polymères peuvent être notamment choisis parmi :
- les sels de l'acide polyvinylsulfonique ayant un poids moléculaire moyen en poids allant de environ 1.000 à 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone.
- les sels de l'acide polystyrène sulfonique les sels de sodium ayant un poids moléculaire moyen en poids d'environ 500.000 et d'environ 100.000 vendus respectivement sous les dénominations Flexan 500 et Flexan 130 par National Starch. Ces composés sont décrits dans le brevet FR 2.198.719.
- les sels d'acides polyacrylamide sulfoniques ceux mentionnés dans le brevet US 4.128.631 et plus particulièrement l'acide polyacrylamidoéthylpropane sulfonique vendu sous la dénomination COSMEDIA POLYMER HSP 1180 par Henkel.

Selon l'invention, les polymères filmogènes anioniques sont de préférence choisis parmi les copolymères d'acide acrylique tels que le terpolymère acide acrylique / acrylate d'éthyle / N-tertiobutylacrylamide vendu sous la dénomination ULTRAHOLD STRONG par la société BASF, les copolymères dérivés d'acide crotonique tels que les terpolymères acétate de vinyle / tertio-butyl benzoate de vinyle / acide crotonique et les terpolymères acide crotonique / acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléique mono estérifié vendu sous la dénomination GANTREZ ES 425 par la société ISP, les copolymères d'acide méthacrylique et de méthacrylate de méthyle vendus sous la dénomination EUDRAGIT L par la société ROHM PHARMA, le copolymère d'acide méthacrylique et d'acrylate d'éthyle vendu sous la dénomination LUVIMER MAEX ou MAE par la société BASF, le copolymère acétate de vinyle/acide crotonique vendu sous la dénomination LUVISET CA 66 par la société BASF et le copolymère acétate de vinyle/acide crotonique greffé par du polyéthylèneglycol sous la dénomination ARISTOFLEX A par la société BASF.

Les polymères filmogènes anioniques les plus particulièrement préférés sont choisis parmi le copolymère méthylvinyléther / anhydride maléique mono estérifié vendu sous la dénomination GANTREZ ES 425 par la société ISP, le terpolymère acide acrylique / acrylate d'éthyle / N-tertiobutylacrylamide vendu sous la dénomination ULTRAHOLD STRONG par la société BASF, les copolymères d'acide méthacrylique et de méthacrylate de méthyle vendus sous la dénomination EUDRAGIT L par la société ROHM PHARMA, les terpolymères acétate de vinyle / tertio-butyl benzoate de vinyle / acide crotonique et les terpolymères acide crotonique / acétate de vinyle / néododécanoate de vinyle vendus sous la dénomination Résine 28-29-30 par la société NATIONAL STARCH, le copolymère d'acide méthacrylique et d'acrylate d'éthyle vendu sous la dénomination LUVIMER MAEX OU MAE par la société BASF, le terpolymère de vinylpyrrolidone / acide acryllque/méthacrylate de lauryle vendu sous la dénomination ACRYLIDONE LM par la société ISP.

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
   Le composé vinylique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
      Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
      Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
      Les comonomères basiques préférés sont des méthacrylates d'aminoéthyle, de butyl aminoéthyle, de N,N'-diméthylaminoéthyle, de N-tertio-butylaminoéthyle.
      On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) Les polyaminoamides réticulés et alcoylés partiellement ou totalement dérivant de polyaminoamides de formule générale :

   ⁅CO―R₄―CO―Z⁆ (III)

   dans laquelle R₄ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 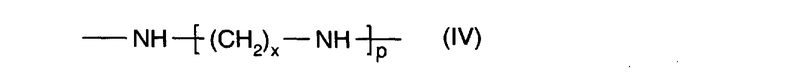 où x=2 et p=2 ou 3, ou bien x=3 et p=2
      ce radical dérivant de la diéthylène triamine, de la triéthylène tétraamine·ou de la dipropylène triamine;
   b) dans les proportions de 0 à 40 moles % le radical (IV) ci-dessus, dans lequel x=2 et p=1 et qui dérive de l'éthylènediamine, ou le radical dérivant de la pipérazine : 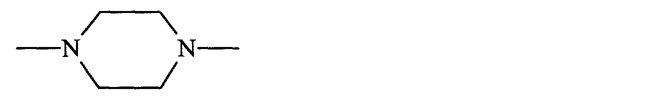
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
      Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 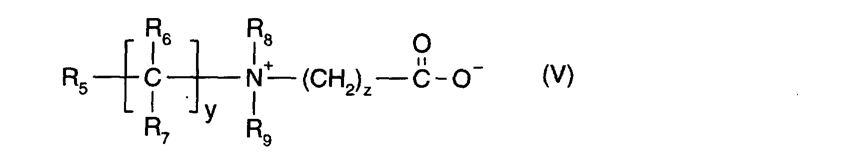 dans laquelle R₅ désigne un groupement insaturé polymérisable tel qu'un groupement acrylate, méthacrylate, acrylamide ou méthacrylamide, y et z représentent un nombre entier de 1 à 3, R₆ et R₇ représentent un atome d'hydrogène, méthyle, éthyle ou propyle, R₈ et R₉ représentent un atome d'hydrogène ou un radical alkyle de telle façon que la somme des atomes de carbone dans R₈ et R₉ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle/méthacrylate de diméthyl-carboxyméthylammonio-éthyl tel que le produit vendu sous la dénomination DIAFORMER Z301 par la société SANDOZ.
(5) les polymères dérivés du chitosane comportant des motifs monomères répondant aux formules (VI), (VII), (VIII) suivantes : 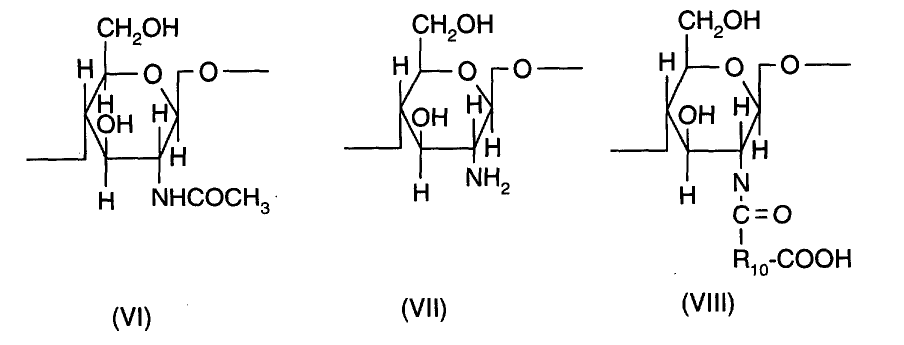 le motif (VI) étant présent dans des proportions allant de 0 à 30%, le motif (VII) dans des proportions allant de 5 à 50% et le motif (VIII) dans des proportions allant de 30 à 90%, étant entendu que dans ce motif (VIII), R₁₀ représente un radical de formule : 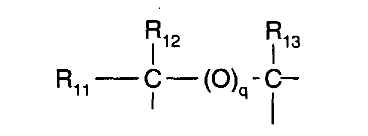 dans laquelle
   si q=0, R₁₁, R₁₂ et R₁₃, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₁₁, R₁₂ et R₁₃ étant dans ce cas un atome d'hydrogène ;
   ou si q=1, R₁₁, R₁₂ et R₁₃ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (IX) tels que ceux décrits par exemple dans le brevet français 1 400 366 : 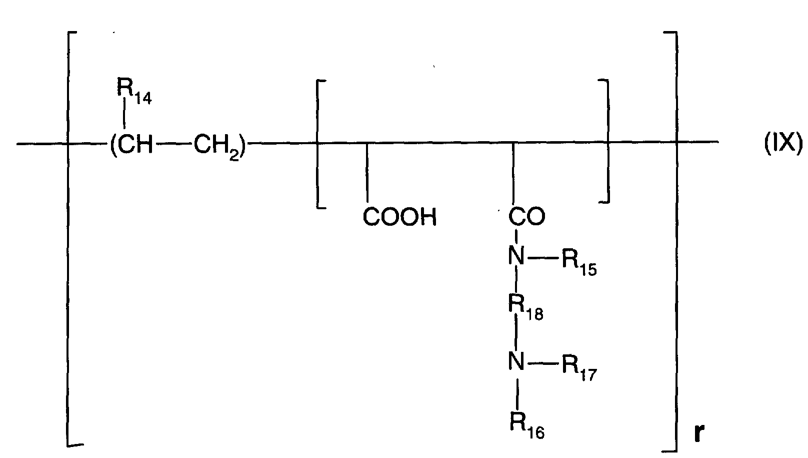 dans laquelle R₁₄ représente un atome d'hydrogène, un radical CH₃O, CH₃CH₂O, phényle, R₁₅ désigne l'hydrogène ou un radical alkyle inférieur tel que méthyle, éthyle, R₁₆ désigne l'hydrogène ou un radical alkyle inférieur tel que méthyle, éthyle, R₁₇ désigne un radical alkyle inférieur tel que méthyle, éthyle ou un radical répondant à la formule : -R₁₈-N(R₁₆)₂, R₁₈ représentant un groupement -CH₂-CH₂- , -CH₂-CH₂-CH₂- , -CH₂-CH(CH₃)- , R₁₆ ayant les significations mentionnées ci-dessus,
   ainsi que les homologues supérieurs de ces radicaux et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (X)

      où D désigne un radical 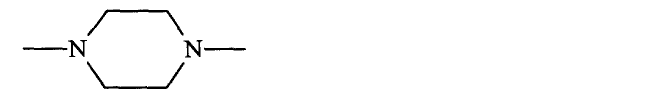 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- (XI)
   où D désigne un radical 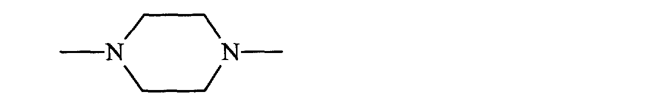 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Les polymères non ioniques utilisables selon la présente invention sont choisis par exemple parmi :
- les homopolymères de vinyipyrrolidone ;
- les copolymères de vinylpyrrolidone et d'acétate de vinyle ;
- les polyalkyloxazolines telles que les polyéthyloxazolines proposées par la société DOW CHEMICAL sous les dénominations PEOX 50000, PEOX 200000 et PEOX 500 000 ;
- les homopolymères d'acétate de vinyle tels que le produit proposé sous le nom de APPRETAN EM par la société HOECHST ou le produit proposé sous le nom de RHODOPAS A 012 par la société RHONE POULENC ;
- les copolymères d'acétate de vinyle et d'ester acrylique tels que le produit proposé sous le nom de RHODOPAS AD 310 de RHONE POULENC ;
- les copolymères d'acétate de vinyle et d'éthylène tels que le produit proposé sous le nom de APPRETAN TV par la société HOECHST ;
- les copolymères d'acétate de vinyle et d'ester maléïque par exemple de maléate de dibutyle tels que le produit proposé sous le nom de APPRETAN MB EXTRA par la société HOECHST ;
- les copolymères de polyéthylène et d'anhydride maléïque ;
- les homopolymères d'acrylates d'alkyle et les homopolymères de méthacrylates d'alkyle tels que le produit proposé sous la dénomination MICROPEARL RQ 750 par la société MATSUMOTO ou le produit proposé sous la dénomination LUHYDRAN A 848 S par la société BASF ;
- les copolymères d'esters acryliques tels que par exemple les copolymères d'acrylates d'alkyle et de méthacrylates d'alkyles tels que les produits proposés par la société ROHM&HAAS sous les dénominations PRIMAL AC-261 K et EUDRAGIT NE 30 D, par la société BASF sous les dénominations ACRONAL 601, LUHYDRAN LR 8833 ou 8845, par la société HOECHST sous les dénominations APPRETAN N 9213 ou N9212;
- les copolymères d'acrylonitrile et d'un monomère non ionique choisi par exemple parmi le butadiène et les (méth)acrylates d'alkyle ; on peut citer les produits proposés sous les dénominations NIPOL LX 531 B par la société NIPPON ZEON ou ceux proposés sous la dénomination CJ 0601 B par la société ROHM & HAAS ;
- les polyuréthannes tels que les produits proposés sous les dénominations ACRYSOL RM 1020 ou ACRYSOL RM 2020 par la société ROHM & HAAS, les produits URAFLEX XP 401 UZ, URAFLEX XP 402 UZ par la société DSM RESINS ;
- les copolymères d'acrylate d'alkyle et d'uréthanne tels que le produit 8538-33 par la société NATIONAL STARCH ;
- les polyamides tels que le produit ESTAPOR LO 11 proposé par la société RHONE POULENC.
- les gommes de guar non ioniques chimiquement modifiées ou non modifiées. Les gommes de guar non ioniques non modifiées sont par exemple les produits vendus sous la dénomination VIDOGUM GH 175 par la société UNIPECTINE et sous la dénomination JAGUAR C par la société MEYHALL.

Les gommes de guar non-ioniques modifiées utilisables selon l'invention sont de préférence modifiées par des groupements hydroxyalkyle en C₁-C ₆. On peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société MEYHALL, ou sous la dénomination GALACTASOL 4H4FD2 par la société AQUALON.

Les radicaux alkyle des polymères non ioniques ont de 1 à 6 atomes de carbone sauf mention contraire.

On peut aussi utiliser, comme polymères, des polyuréthannes fonctionnalisés ou non.

Les polyuréthannes particulièrement visés par la présente invention sont ceux décrits dans les brevets EP 0 751 162, EP 0 637 600, FR 2 743 297 et EP 0 648 485 dont la Demanderesse est Titulaire, ainsi que le brevets EP 0 656 021 ou WO 94/03510 de la Société BASF et EP 0 619 111 de la Société National Starch.

Les agents conditionneurs de type polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre allant de 500 à 5.10⁶ environ, et de préférence allant de 10³ à 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 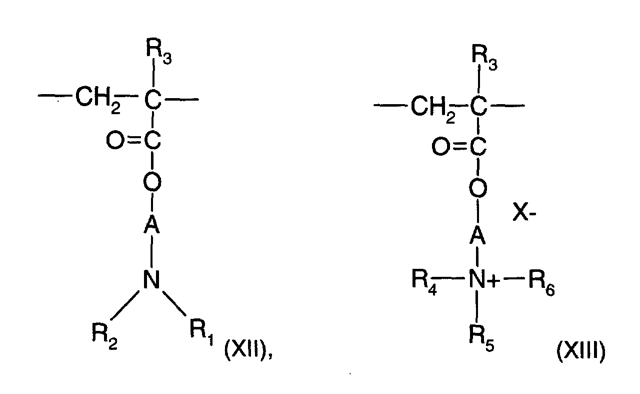 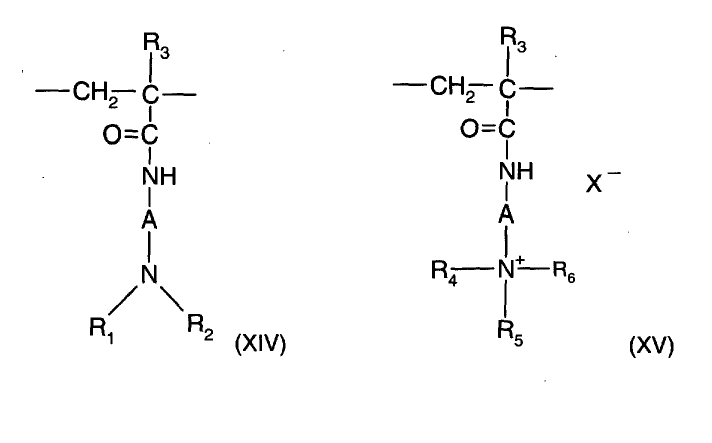 dans lesquelles:
   R₁ et R₂, identiques ou différents, représentent hydrogène ou un groupe alkyle ayant de 1 à 6 atomes de carbone et de préférence méthyle ou éthyle;
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   X désigne un anion dérivé d'un acide minéral ou organique tel que un anion méthosulfate ou un halogénure tel que chlorure ou bromure.
   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/vinylpyrrolidone tel que le produit vendu sous la dénomination GAFFIX VC 713 par la société ISP,
   - les copolymères vinylpyrrolidone / méthacrylamidopropyl dimethylamine commercialisés notamment sous la dénomination STYLEZE CC 10 par ISP.
   - et les copolymères vinylpyrrolidone / méthacrylamide de diméthylaminopropyle quaternisé tel que le produit vendu sous la dénomination "GAFQUAT HS 100" par la société ISP.
2) Les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.
   Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
   Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
   Les gommes de galactomannane cationiques sont décrites plus particulièrement dans les brevets US 3 589 578 et 4 031 307 en particulier les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(3) les polymères comprenant de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipiquediacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant allant de 0,8 : 1 à 1,4 : 1; le polyaminoamide en résultant étant amené à réagir avec l'épichlorhydrine dans un rapport molaire d'épichlorhydrine par rapport au groupement amine secondaire du polyaminoamide allant de 0,5 : 1 à 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(7) les cyclopolymères d'alkyl diallyl amine ou de dialkyl diallyl ammonium tels que les homopolymères ou copolymères comportant comme constituant principal de la chaîne des motifs répondant aux formules (XVI) ou (XVII) : 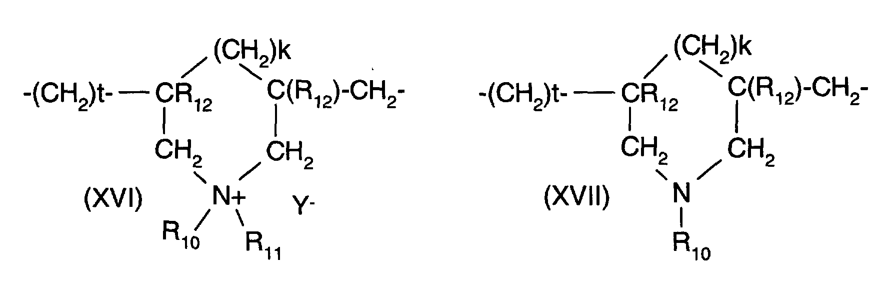 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faibles masses moléculaires moyenne en poids) et les copolymères de chlorure de diallyldiméthylammonium et d'acrylamide commercialisés sous la dénomination "MERQUAT 550".
(8) les polymères de diammonium quaternaire contenant des motifs récurants répondant à la formule : 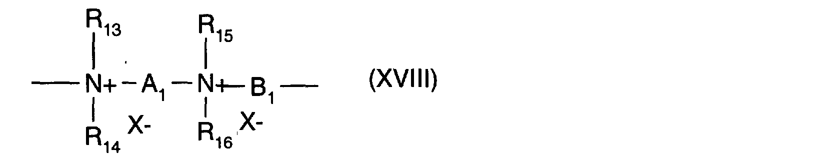 formule (XVIII) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₃ et R₁₅ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH₂)ₙ-CO-D-OC-(CH₂)n-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH₂-CH₂-O)ₓ-CH₂-CH₂-

      -[CH₂-CH(CH₃)-O]_{y}-CH₂-CH(CH₃)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH₂-CH₂-S-S-CH₂-CH₂- ;
   d) un groupement uréylène de formule : -NH-CO-NH- ;

   De préférence, X⁻ est un anion tel que le chlorure ou le bromure.
   Ces polymères ont une masse moléculaire moyenne en nombre généralement allant de 1000 à 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui comprennent des motifs récurrents répondant à la formule : 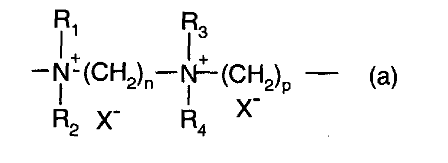 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   Un composé de formule (a) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).
(9) les polymères de polyammonium quaternaires comprenant des motifs de formule (XIX): 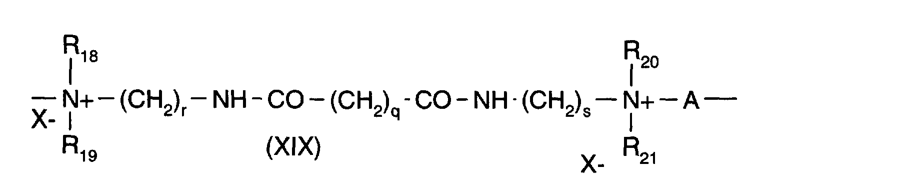 formule dans laquelle :
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier allant de 1 à 6, sous réserve que R₁₈, R₁₉, R₂₀ et R₂₁ ne représentent pas simultanément un atome d'hydrogène,
   r et s, identiques ou différents, sont des nombres entiers allant de 1 à 6,
   q est égal à 0 ou à un nombre entier allant de 1 à 34,
   X- désigne un anion tel qu'un halogènure,
   A désigne un radical d'un dihalogénure ou représente de préférence -CH₂-CH₂-O-CH₂-CH₂-.
   De tels composés sont notamment décrits dans la demande de brevet EP-A-122 324.
   On peut par exemple citer parmi ceux-ci, les produits "Mirapol® A 15", "Mirapol® AD1", "Mirapol® AZ1" et "Mirapol® 175" vendus par la société Miranol.
(10) Les polymères quaternaires de vinylpyrrolidone et de vinylimidazole tels que par exemple les produits commercialisés sous les dénominations Luviquat® FC 905, FC 550 et FC 370 par la société B.A.S.F.
(11) Les polyamines comme le Polyquart® H vendu par HENKEL, référencé sous le nom de « POLYETHYLENEGLYCOL (15) TALLOW POLYAMINE » dans le dictionnaire CTFA.
(12) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE® SC 95 » et « SALCARE® SC 96 » par la Société ALLIED COLLOIDS.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la Société UNION CARBIDE CORPORATION, les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société CALGON, les polymères quaternaires de vinylpyrrolidone et de vinylimidazole et leurs mélanges.

Les protéines ou hydrolysats de protéines cationiques sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :
- les hydrolysats de collagène portant des groupements triéthylammonium tels que les produits vendus sous la dénomination "Quat-Pro E" par la Société MAYBROOK et dénommés dans le dictionnaire CTFA "Triéthonium Hydrolyzed Collagen Ethosulfate" ;
- les hydrolysats de collagène portant des groupements chlorure de triméthylammonium et de triméthylstéarylammonium, vendus sous la dénomination de "Quat-Pro S" par la Société MAYBROOOK et dénommés dans le dictionnaire CTFA "Steartrimonium Hydrolyzed Collagen" ;
- les hydrolysats de protéines animales portant des groupements triméthylbenzylammonium tels que les produits vendus sous la dénomination "Crotein BTA" par la Société CRODA et dénommés dans le dictionnaire CTFA "Benzyltrimonium hydrolyzed animal protein" ;
- Ies hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres :
- le "Croquat L" dont les groupements ammonium quaternaires comportent un groupement alkyle en C₁₂ ;
- le "Croquat M" dont les groupements ammonium quaternairees comportent des groupements alkyle en C₁₀-C₁₈ ;
- le "Croquat S" dont les groupements ammonium quaternaires comportent un groupement alkyle en C₁₈ ;
- le "Crotein Q" dont les groupements ammonium quaternaires comportent au moins un groupe alkyle ayant de 1 à 18 atomes de carbone.
Ces différents produits sont vendus par la Société Croda.

D'autres protéines ou hydrolysats quaternisés sont par exemple ceux répondant à la formule : 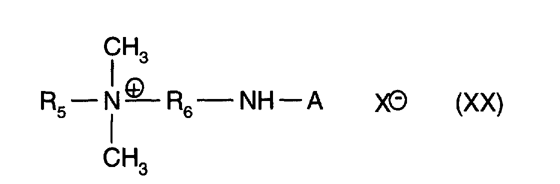 dans laquelle X⁻ est un anion d'un acide organique ou minéral, A désigne un reste de protéine dérivé d'hydrolysats de protéine notamment de collagène, R₅ désigne un groupement lipophile comportant jusqu'à 30 atomes de carbone, R₆ représente un groupement alkylène ayant 1 à 6 atomes de carbone. On peut citer par exemple les produits vendus par la Société Inolex, sous la dénomination "Lexein QX 3000", appelé dans le dictionnaire CTFA "Cocotrimonium Collagent Hydrolysate".

On peut encore citer les protéines végétales quaternisées telles que les protéines de blé, de maïs ou de soja : comme protéines de blé quaternisées, on peut citer celles commercialisées par la Société Croda sous les dénominations "Hydrotriticum WQ ou QM", appelées dans le dictionnaire CTFA "Cocodimonium Hydrolysed wheat protein", "Hydrotriticum QL" appelée dans le dictionnaire CTFA "Laurdimonium hydrolysed wheat protein", ou encore "Hydrotriticum QS", appelée dans le dictionnaire CTFA "Steardimonium hydrolysed wheat protein".

Les sels d'ammonium quaternaires comportant au moins une fonction amide peuvent être choisis parmi ceux qui présentent la formule générale (XXI) suivante : 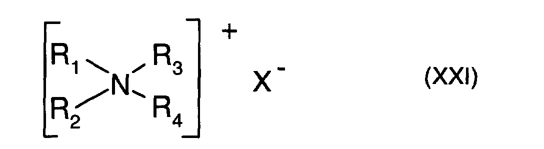 dans laquelle les radicaux R₁ à R₄, qui peuvent être identiques ou différents, sont choisis parmi les radicaux alkyle ayant de 1 à 30 atomes de carbone, polyoxyalkylène(C₂-C₆), alkyl(C₁₂-C₂₂)amido alkyle(C₂-C₆), alkyl(C₁₂-C₂₂)acétate, hydroxyalkyle comportant environ de 1 à 30 atomes de carbone; X est un anion cosmétiquement acceptable par exemple choisi dans le groupe des halogénures, phosphates, acétates, lactates, alkyl(C₂-C₆)sulfates, alkyl-ou-alkylarylsulfonates, sous réserve qu'au moins un des radicaux contiennent au moins une fonction amide.

Parmi les sels d'ammonium quaternaire de formule (XXI), on préfère le chlorure de palmitylamidopropyl triméthylammonium ou le chlorure de stéaramidopropyldiméthyl(myristyl acétate)-ammonium commercialisé sous la dénomination CERAPHYL® 70 par la société VAN DYK.

Les sels d'ammonium quaternaire de l'imidazolinium, sont notamment choisis parmi ceux de formule (XXII) suivante : 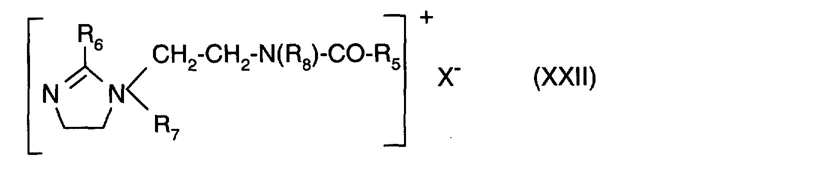 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple commercialisé sous la dénomination «REWOQUAT W 75» par la société REWO,
- Les sels de diammonium quaternaire sont notamment choisis parmi ceux de formule (XXIII) : 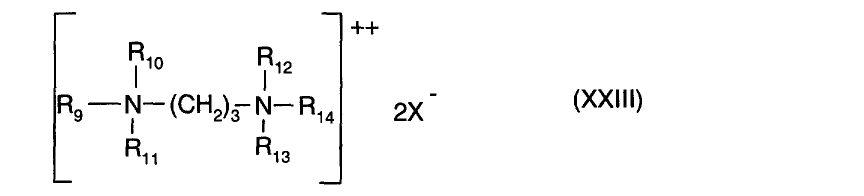 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.

Les sels d'ammonium quaternaire contenant au moins une fonction ester utilisables selon l'invention sont par exemple ceux de formule (XXIV ) suivante : 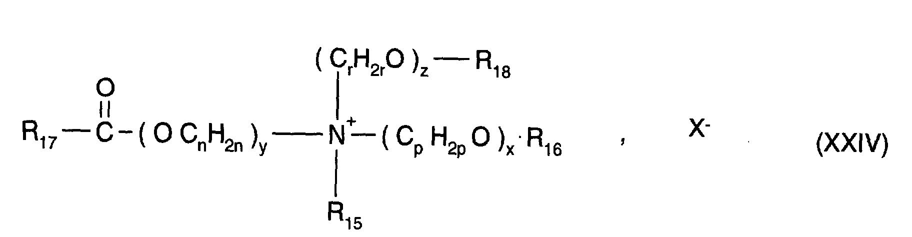 dans laquelle :
- R₁₅ est choisi parmi les radicaux alkyles en C₁-C₆ et les radicaux hydroxyalkyles ou dihydroxyalkyles en C₁-C₆;
- R₁₆ est choisi parmi :
   - le radical 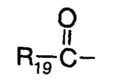
   - les radicaux R₂₀ hydrocarbonés en C₁-C₂₂ linéaires ou ramifiés, saturés ou insaturés,
   - l'atome d'hydrogène,
- R₁₈ est choisi parmi :
   - le radical 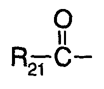
   - les radicaux R₂₂ hydrocarbonés en C₁-C₆ linéaires ou ramifiés, saturés ou insaturés,
   - l'atome d'hydrogène,
- R₁₇, R₁₉ et R₂₁, identiques ou différents, sont choisis parmi les radicaux hydrocarbonés en C₇-C₂₁, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X⁻ est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

Les radicaux alkyles R₁₅ peuvent être linéaires ou ramifiés et plus particulièrement linéaires.

De préférence R₁₅ désigne un radical méthyle, éthyle, hydroxyéthyle ou dihydroxypropyle et plus particulièrement un radical méthyle ou éthyle.

Avantageusement, la somme x + y + z vaut de 1 à 10.

Lorsque R₁₆ est un radical R₂₀ hydrocarboné, il peut être long et avoir de 12 à 22 atomes de carbone ou court et avoir de 1 à 3 atomes de carbone.

Lorsque R₁₈ est un radical R₂₂ hydrocarboné, il a de préférence 1 à 3 atomes de carbone.

Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.
Avantageusement, y est égal à 1.
De préférence, n, p et r, identiques ou différents, valent 2 ou 3 et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement les sels d'ammonium de formule (XXIV) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 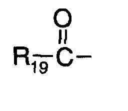
   - les radicaux méthyle, éthyle ou hydrocarbonés en C₁₄-C₂₂
   - l'atome d'hydrogène ;
- R₁₈ est choisi parmi :
   - le radical 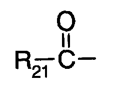
   - l'atome d'hydrogène ;
R₁₇, R₁₉ et R₂₁, identiques ou différents, sont choisis parmi les radicaux hydrocarbonés en C₁₃-C₁₇, linéaires ou ramifiés, saturés ou insaturés et de préférence parmi les radicaux alkyles et alcényle en C₁₃-C₁₇, linéaires ou ramifiés, saturés ou insaturés.
Avantageusement, les radicaux hydrocarbonés sont linéaires.

On peut citer par exemple les composés de formule (XXIV) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus par exemple par estérification directe de la triéthanolamine, de la triisopropanolamine, d'alkyldiéthanolamine ou d'alkyidiisopropanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART par la société HENKEL, STEPANQUAT par la société STEPAN, NOXAMIUM par la société CECA, REWOQUAT WE 18 par la société REWO-WITCO.

La composition selon l'invention contient de préférence un mélange de sels de mono, di et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl dihydroxyéthyl méthyl ammonium, 45 à 60% de méthylsulfate de diacyloxyéthyl hydroxyéthyl méthyl ammonium et 15 à 30% de méthylsulfate de triacyloxyéthyl méthyl ammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Selon l'invention, le ou les agents conditionneurs de préférence solubles selon l'invention peuvent représenter de 0,001 % à 20 % en poids, de préférence de 0,01 % à 10% en poids et plus particulièrement de 0,05 à 3% en poids par rapport au poids total de la composition finale.

Les compositions de l'invention contiennent en outre avantageusement au moins un autre agent tensioactif qui est généralement présent en une quantité allant de 0,1% à 40% en poids environ, de préférence allant de 3% à 30% et encore plus préférentiellement allant de 5% à 20%, par rapport au poids total de la composition.

Cet agent tensioactif peut être choisi parmi les agents tensioactifs anioniques, amphotères, non-ioniques ou leurs mélanges.

Les tensioactifs additionnels convenant à la mise en oeuvre de la présente invention sont notamment les suivants :

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersulfates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères additionnels, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle :
B représente -CH₂CH₂OX', C représente -(CH₂)_{z}-Y', avec z = 1 ou 2,
X' désigne le groupement -CH₂CH₂-COOH ou un atome d'hydrogène
Y' désigne -COOH ou le radical -CH₂- CHOH - SO₃H
R₅ désigne un radical alkyle d'un acide carboxylique présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 7ème édition, 1997, sous les dénominations Disodium Cocoamphodiacetate, Disodium Lauroamphodiacetate, Disodium Capryloamphodiacetate, Disodium Caproamphodiacetate, Disodium Cocoamphodipropionate, Disodium Lauroamphodipropionate, Disodium Caproamphodipropionate, Disodium Capryloamphodipropionate, Lauroamphodipropionic acid, Cocoamphodipropionic acid.
A titre d'exemple on peut citer le disodium cocoamphodiacetate commercialisé sous la dénomination commerciale MIRANOL® C2M concentré par la société RHODIA CHIMIE.

Dans les compositions conformes à l'invention, on utilise de préférence des mélanges d'agents tensioactifs et en particulier des mélanges d'agents tensioactifs anioniques ou des mélanges d'agents tensioactifs anioniques et d'agents tensioactifs amphotères ou non ioniques.

On utilise de préférence comme agent tensioactif anionique additionnel les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélanges avec :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL® C2M CONC" en solution aqueuse à 38 % de matière active ou sous la dénomination MIRANOL® C32;
- soit un agent tensioactif amphotère tel que les alkylbétaïnes en particulier la cocobétaïne commercialisée sous la dénomination "DEHYTON® AB 30" en solution aqueuse à 32 % de MA par la société HENKEL ou tel que les alkyl (C₈-C₂₀) amidoalkyl (C₁-C₆) bétaïnes en particulier la TEGOBETAINE® F 50 commercialisée par la société GOLDSCHMIDT.

Le(s) agent(s) tensioactif(s) anionique(s) différents des composés N-acylés d'aminoacides polycarcarboxyliques mono ou polyamidifiés selon l'invention sont généralement présents à raison de 1 à 30 % en poids, de préférence de 3 à 15 % en poids, par rapport au poids total de la composition.

Le(s) agent(s) tensioactif(s) amphotère(s) ou non ioniques sont généralement présents à raison de 0,5 à environ 15% en poids, de préférence de 1 à 5% en poids, par rapport au poids total de la composition.

La quantité et la qualité des tensioactifs sont celles suffisantes pour conférer à la composition finale un pouvoir moussant et/ou détergent satisfaisant.

Dans la composition selon la présente invention, la totalité des tensioactifs détergents représente généralement de 4 à 50% en poids et de préférence de 6 à 35% en poids et plus particulièrement de 8 à 25% en poids par rapport au poids total de la composition.

La composition de l'invention peut également contenir au moins un additif choisi parmi les épaississants, les parfums, les agents nacrants, les conservateurs, les filtres solaires, les silicones, les céramides, les pseudocéramides, les acides gras à chaînes linéaires ou ramifiées en C₁₆-C₄₀ tels que l'acide méthyl-18 eicosanoique, les hydroxyacides, les vitamines, les provitamines telles que le panthénol, les huiles végétales, les huiles minérales et les huiles de synthèse, les agents antipelliculaires et tout autre additif classiquement utilisé dans le domaine cosmétique qui n'affecte pas la stabilité et les propriétés des compositions selon l'invention.

Ces additifs sont éventuellement présents dans la composition selon l'invention dans des proportions pouvant aller de 0,0001 à 50% en poids par rapport au poids total de la composition. La quantité précise de chaque additif est déterminée facilement par l'homme du métier selon sa nature et sa fonction.

Le milieu cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol ; les alkylèneglycols comme le propylèneglycol, les éthers de glycols.
De préférence, la composition comprend de 50 à 95 % en poids d'eau par rapport au poids total de la composition.

Les compositions détergentes selon l'invention présentent un pH final généralement allant de 3 à 10. De préférence, ce pH est allant de 4 à 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions selon l'invention peuvent contenir également des synergistes de mousses tels que des 1,2-alcanediols en C₁₀-C₁₈ ou des alcanolamides gras dérivés de mono ou de diéthanolamine.

Les compositions conformes à l'invention peuvent être utilisées pour le lavage et le traitement des matières kératiniques telles que les cheveux, la peau, les cils, les sourcils, les ongles, les lèvres, le cuir chevelu et plus particulièrement les cheveux.

En particulier, les compositions détergentes selon l'invention sont des shampooings, des gels-douche et des bains moussants.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de produits démaquillants.

Les compositions selon l'invention peuvent se présenter sous forme de gel, de lait, de crème, d'émulsion, de lotion épaissie ou de mousse et être utilisées pour la peau, le cuir chevelu, les ongles, les cils, les lèvres et plus particulièrement les cheveux.

Ces compositions détergentes sont de préférence moussantes et le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes :
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombent dans une éprouvette ayant un diamètre de 50 mm et contenant 50 ml de la composition à tester. La mesure est faite 5 minutes après l'arrêt de l'écoulement de la composition.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage en particulier avec de l'eau.

Ainsi, ce procédé selon l'invention permet le traitement, le soin, le lavage ou le démaquillage de la peau, des cheveux ou de toute autre matière kératinique.

Dans tout ce qui suit ou ce qui précède, les pourcentages exprimés sont en poids.
L'invention va être maintenant plus complètement illustrée à l'aide des exemples suivants qui ne sauraient être considérés comme la limitant aux modes de réalisation décrits. Dans les exemples, MA signifie matière active.

### EXEMPLE 1 :

On a réalisé trois compositions de shampooings, l'une conforme à l'invention (composition A) et les 2 autres comparatives (compositions B et C) :

| | A (invention) | B | C |
|---|---|---|---|
| - Sel de triéthanolamine de N-cocoyl glutamine à 30%MA (Foam up doucé GM de KYOWA HAKKO) | 16,7 g (5 gMA) | ― | ― |
| - Cocoylbétaine en solution aqueuse à 30%MA (DEHYTON AB 30 de COGNIS) | 8,3 g (2,5 gMA) | 8,3 g (2,5 gMA) | 8,3 g (2,5 gMA) |
| -Alkyl(C₁₂-C₁₄)éther sulfate de sodium oxyéthyléné à 2,2 moles d'oxyde d'éthylène en solution aqueuse à 26% de MA | 38 g (9,9 gMA) | 38 g (9,9 gMA) | 57 g (14,8 gMA) |
| N-cocoyl glutamate en solution aqueuse à 30% MA (Acyl glutamate CT12 de AJINOMOTO) | ― | 5 gMA | ― |
| - Hydroxyéthylcellulose quaternisée par du 2,3-époxypropyl triméthyl ammonium (JR400 de AMERCHOL) | 1 g | 1 g | 1 g |
| - Conservateurs | qs | qs | qs |
| Agent de pH qs pH | 6,5 | 6,5 | 6,5 |
| - Eau déminéralisée qsp | 100 g | 100 g | 100 g |

On effectue un shampooing en appliquant environ 1 g de la composition A sur des mèches de 2,5 g de cheveux sensibilisés préalablement mouillés. On fait mousser le shampooing, on laisse pauser pendant 10 minutes puis on rince abondamment à l'eau. On sèche les mèches pendant 10 minutes à 60°C. On fait une deuxième application de la composition.
On procède selon le même mode opératoire que ci-dessus avec les compositions comparatives B et C.

Un panel d'experts a évalué l'aspect des cheveux mouillés.
100% des experts indiquent que les cheveux traités avec la composition A selon l'invention sont significativement plus lisses, plus doux que ceux traités avec les compositions B et C.

### EXEMPLE 2 :

On a réalisé deux compositions de shampooings, l'une conforme à l'invention (composition D) et l'autre comparative (composition E ) :

| | D (invention) | E |
|---|---|---|
| - Sel de triéthanolamine de N-cocoyl glutamine à 30%MA (Foam up doucé GM de KYOWA HAKKO) | 16,7 g (5 gMA) | ― |
| - Cocoylbétaine en solution aqueuse à 30%MA (DEHYTON AB 30 de COGNIS) | 8,3 g (2,5 gMA) | 8,3 g (2,5 gMA) |
| - Alkyl(C₁₂-C₁₄)éther sulfate de sodium oxyéthyléné à 2,2 moles d'oxyde d'éthylène en solution aqueuse à 26% de MA | 38 g (9,9 gMA) | 57 g (14,8 gMA) |
| - Hydroxyéthyl- cellulose greffée avec un sel de méthacryloyléthyl triméthylammonium (CELQUAT de NATIONAL STARCH) | 1 g | 1 g |
| - Conservateurs | qs | qs |
| Agent de pH qs pH | 6,5 | 6,5 |
| - Eau déminéralisée qsp | 100 g | 100 g |

On effectue un shampooing en appliquant environ 1 g de la composition D sur des mèches de 2,5 g de cheveux sensibilisés préalablement mouillés. On fait mousser le shampooing, on laisse pauser pendant 10 minutes puis on rince abondamment à l'eau. On sèche les mèches pendant 10 minutes à 60°C. On fait une deuxième application de la composition.
On procède selon le même mode opératoire que ci-dessus avec la composition comparative E.

Un panel d'experts a évalué l'aspect des cheveux mouillés.
100% des experts indiquent que les cheveux traités avec la composition D selon l'invention sont significativement plus lisses et se démêlent facilement que ceux traités avec la composition E.

### EXEMPLE 3 :

On a réalisé deux compositions de shampooings, l'une conforme à l'invention (composition F) et l'autre comparative (composition G ) :

| | F (invention) | G |
|---|---|---|
| - Sel de triéthanolamine de N-cocoyl glutamine à 30%MA (Foam up doucé GM de KYOWA HAKKO) | 16,7 g (5 gMA) | ― |
| - Cocoylbétaine en solution aqueuse à 30%MA (DEHYTON AB 30 de COGNIS) | 8,3 g (2,5 gMA) | 8,3 g (2,5 gMA) |
| - Alkyl(C₁₂-C₁₄)éther sulfate de sodium oxyéthyléné à 2,2 moles d'oxyde d'éthylène en solution aqueuse à 26% de MA | 38 g (9,9 gMA) | 57 g (14,8 gMA) |
| - Polyurethane-1 (LUVISET PUR de BASF) | 8,3 g (2,5 gMA) | 8,3 g (2,5 gMA) |
| - Conservateurs | qs | qs |
| Agent de pH qs pH | 6,5 | 6,5 |
| - Eau déminéralisée qsp | 100 g | 100 g |

On effectue un shampooing en appliquant environ 1 g de la composition F sur des mèches de 2,5 g de cheveux sensibilisés préalablement mouillés. On fait mousser le shampooing, on laisse pauser pendant 10 minutes puis on rince abondamment à l'eau. On sèche les mèches pendant 10 minutes à 60°C. On fait une deuxième application de la composition.
On procède selon le même mode opératoire que ci-dessus avec la composition comparative G.

Un panel d'experts a évalué l'aspect des cheveux mouillés.
90% des experts indiquent que les cheveux traités avec la composition F selon l'invention sont significativement plus lisses et/ou plus doux et/ou se démêlent facilement que ceux traités avec la composition G.

### EXEMPLE 4 :

On a réalisé deux compositions de shampooings, l'une conforme à l'invention (composition H) et l'autre comparative (composition I) :

| | H (invention) | I |
|---|---|---|
| - Sel de triéthanolamine de N-cocoyl glutamine à 30%MA (Foam up doucé GM de KYOWA HAKKO) | 16,7 g (5,01 gMA) | ― |
| - Cocoylbétaine en solution aqueuse à 30%MA (DEHYTON AB 30 de COGNIS) | 8,3 g (2,49 gMA) | 8,3 g (2,49 gMA) |
| -Alkyl(C₁₂-C₁₄)éther sulfate de sodium oxyéthyléné à 2,2 moles d'oxyde d'éthylène en solution aqueuse à 26% de MA | 38 g (9,88 gMA) | 57 g (14,82 gMA) |
| - Copolymère de méthacryloyléthyl N,N-diméthyl carboxyméthyl bétaïne et de méthacrylate de méthyle en solution aqueuse à 22% de MA | 1 g (0,22 g MA) | 1 g (0,22 g MA) |
| - Conservateurs | qs | qs |
| - Agent de pH qs pH | 6,5 | 6,5 |
| - Eau déminéralisée qsp | 100 g | 100 g |

On effectue un shampooing en appliquant environ 1 g de la composition H sur des mèches de 2,5 g de cheveux sensibilisés préalablement mouillés. On fait mousser le shampooing, on laisse pauser pendant 10 minutes puis on rince abondamment à l'eau. On sèche les mèches pendant 10 minutes à 60°C. On fait une deuxième application de la composition.
On procède selon le même mode opératoire que ci-dessus avec la composition comparative I.

Un panel d'experts a évalué l'aspect des cheveux mouillés.
100% des experts indiquent que les cheveux traités avec la composition H selon l'invention sont significativement plus lisses et/ou plus doux que ceux traités avec la composition I.

## Revendications

1. Composition cosmétique détergente, **caractérisée par le fait qu'**elle comprend, dans un milieu cosmétiquement acceptable, au moins un tensioactif anionique choisi parmi les composés N-acylés d'aminoacides polycarboxyliques mono ou poly amidifiés et leurs sels et au moins un agent conditionneur soluble choisi parmi :
I) les polymères anioniques,
II) les polymères non ioniques,
III) les polymères cationiques,
IV) les polymères amphotères,
V) les protéines cationiques,
VI) les hydrolysats de protéines cationiques et
VII) les tensioactifs cationiques choisis parmi les sels d'ammonium quaternaires comportant au moins une fonction amide, les sels d'ammonium quaternaires de l'imidazoline, les sels de diammonium quaternaires, les sels d'ammonium quaternaire contenant au moins une fonction ester.

2. Composition selon la revendication 1, **caractérisée par le fait que** ledit tensioactif anionique est choisi parmi les composés N-acylés d'aminoacides polycarboxyliques mono ou poly amidifiés et leurs sels de formule suivante (I) : 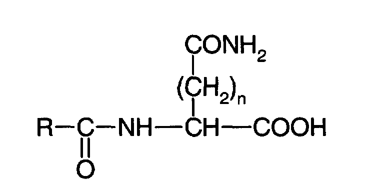 dans laquelle :
R désigne un radical hydrocarboné, saturé ou insaturé, linéaire ou ramifié comportant de 5 à 29 atomes de carbone.
n est égal à 1 ou 2.

3. Composition selon la revendication 2, **caractérisée par le fait que** R désigne un radical alkyle ou alkényle mono ou polyinsaturé comportant de 5 à 29 atomes de carbone et de préférence de 7 à 22 atomes de carbone.

4. Composition selon l'une quelconque des revendications 1 à 3, **caractérisée par le fait que** ledit tensioactif anionique est un sel de N-cocoylglutamine.

5. Composition selon l'une quelconque des revendications précédentes, **caractérisée par le fait que** le polymère anionique est choisi parmi :
- les polymères comportant des motifs carboxyliques dérivant de monomères mono ou diacides carboxyliques insaturés de formule : 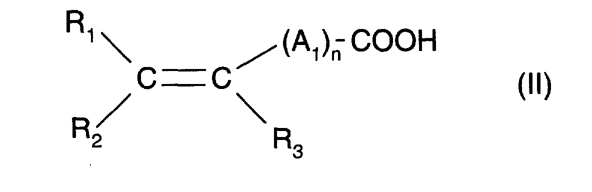 dans laquelle n est un nombre entier de 0 à 10, A₁ désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₁ désigne un atome d'hydrogène, un groupement phényle ou benzyle, R₂ désigne un atome d'hydrogène, un groupement alkyle inférieur ou carboxyle, R₃ désigne un atome d'hydrogène, un groupement alkyle inférieur, un groupement -CH₂-COOH, phényle ou benzyle ;
- les polymères comprenant des motifs dérivant d'acide sulfonique tels que des motifs vinylsulfonique, styrènesulfonique, acrylamido alkylsulfonique.

6. Composition selon la revendication précédente, **caractérisée en ce que** le polymère anionique est choisi parmi :
- les copolymères d'acide acrylique tels que le terpolymère acide acrylique/acrylate d'éthyle/N-tertiobutylacrylamide ;
- les copolymères dérivés d'acide crotonique tels que les terpolymères acétate de vinyle / tertio-butyl benzoate de vinyle / acide crotonique et les terpolymères acide crotoniquelacétate de vlnyle/néododécanoate de vinyle ;
- les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifié ;
- les copolymères d'acide méthacrylique et de méthacrylate de méthyle ;
- le copolymère d'acide méthacrylique et d'acrylate d'éthyle;
- le copolymère acétate de vinyle/acide crotonique ;
- le terpolymère acétate de vinyle/acide crotonique/polyéthylèneglycol.

7. Composition selon l'une quelconque des revendications 1 à 4, **caractérisée en ce que** le polymère amphotère est choisi parmi les polymères comportant des motifs dérivant :
a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

8. Composition selon l'une quelconque des revendications 1 à 4, **caractérisée par le fait que** le polymère non ionique est choisi parmi :
- les polyalkyloxazolines;
- les homopolymères d'acétate de vinyle;
- les copolymères d'acétate de vinyle et d'ester acrylique;
- les copolymères d'acétate de vinyle et d'éthylène;
- les copolymères d'acétate de vinyle et d'ester maléïque;
- les copolymères de polyéthylène et d'anhydride maléïque;
- les homopolymères d'acrylates d'alkyle et les homopolymères de méthacrylates d'alkyle;
- les copolymères d'esters acryliques tels que par exemple les copolymères d'acrylates d'alkyle et de méthacrylates d'alkyle ;
- les copolymères d'acrylonitrile et d'un monomère non ionique choisi par exemple parmi le butadiène et les (méth)acrylates d'alkyle; et
- les copolymères d'acrylate d'alkyle et d'uréthanne.

9. Composition selon l'une quelconque des revendications 1 à 4, **caractérisée par le fait que** les polymères cationiques sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

10. Composition selon la revendication 9, **caractérisée par le fait que** ledit polymère cationique est choisi parmi les cyclopolymères cationiques, les polysaccharides cationiques, les polymères quaternaires de vinylpyrrolidone et de vinylimidazole et leurs mélanges.

11. Composition selon la revendication 10, **caractérisée par le fait que** ledit cyclopolymère est choisi parmi les homopolymères du chlorure de diallyldiméthylammonium et les copolymères du chlorure de diallyldiméthylammonium et d'acrylamide.

12. Composition selon la revendication 10, **caractérisée par le fait que** lesdits polysaccharides cationiques sont choisis parmi les gommes de guar modifiées par un sel de 2,3-époxypropyl triméthylammonium et les dérivés d'éther de cellulose quaternaires, notamment parmi les hydroxyéthylcelluloses ayant réagi avec un époxyde substitué par un groupement triméthylammonium.

13. Composition selon l'une quelconque des revendications 1 à 4, **caractérisée en ce que** les sels d'ammonium quaternaire comportant au moins une fonction amide sont choisis parmi ceux qui présentent la formule générale (XXI) suivante : 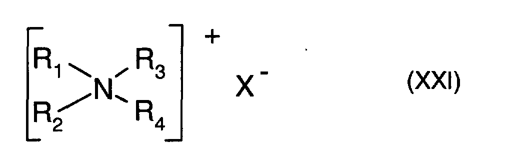 dans laquelle les radicaux R₁ à R₄, qui peuvent être identiques ou différents, sont choisis parmi les radicaux alkyle ayant de 1 à 30 atomes de carbone, polyoxyalkylène(C₂-C₆), alkyl(C₁₂-C₂₂)amido alkyle(C₂-C₆), alkyl(C₁₂-C₂₂)acétate, hydroxyalkyle comportant environ de 1 à 30 atomes de carbone; X est un anion cosmétiquement acceptable par exemple choisi dans le groupe des halogénures, phosphates, acétates, lactates, alkyl(C₂-C₆)sulfates, alkyl-ou-alkylarylsulfonates, sous réserve qu'au moins un des radicaux contienent au moins une fonction amide.

14. Composition selon l'une quelconque des revendications 1 à 4, **caractérisée en ce que** les sels d'ammonium quaternaire de l'imidazoline sont choisisi parmi ceux de formule (XXII) suivante : 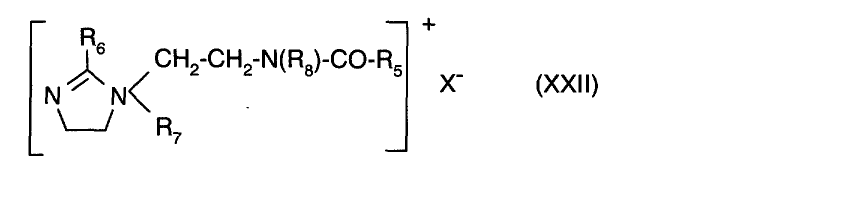 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates.

15. Composition selon l'une quelconque des revendications 1 à 4, **caractérisée en ce que** les sels de diammonium quaternaire sont choisis parmi ceux de formule (XXIII) : 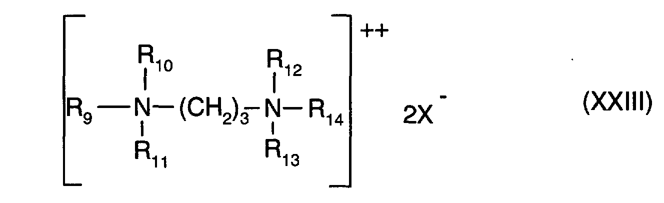 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates.

16. Composition selon l'une quelconque des revendications 1 à 4, **caractérisée en ce que** les sels d'ammonium quaternaire contenant au moins une fonction ester sont choisis parmi ceux de formule (XXIV ) suivante : 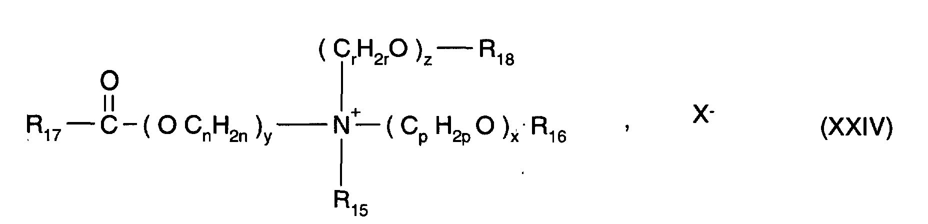 dans laquelle :
- R₁₅ est choisi parmi les radicaux alkyles en C₁-C₆ et les radicaux hydroxyalkyles ou dihydroxyalkyles en C₁-C₆ ;
- R₁₆ est choisi parmi :
- le radical 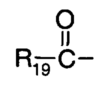
- les radicaux R₂₀ hydrocarbonés en C₁-C₂₂ linéaires ou ramifiés, saturés ou insaturés,
- l'atome d'hydrogène,
- R₁₈ est choisi parmi :
- le radical 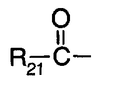
- les radicaux R₂₂ hydrocarbonés en C₁-C₆ linéaires ou ramifiés, saturés ou insaturés,
- l'atome d'hydrogène,
- R₁₇, R₁₉ et R₂₁, identiques ou différents, sont choisis parmi les radicaux hydrocarbonés en C₇-C₂₁, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X⁻ est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

17. Composition selon l'une quelconque des revendications 1 à 16, **caractérisée en ce que** le ou les tensioactifs anioniques choisis parmi les composés N-acylés d'aminoacides polycarboxyliques mono ou poly amidifiés sont présents à une concentration allant de 1 à 30 % en poids, de préférence allant de 3 à 15% en poids par rapport au poids total de la composition.

18. Composition selon l'une quelconque des revendications 1 à 17, **caractérisée en ce que** ledit ou les agents conditionneurs solubles sont présents à une concentration allant de 0,001 % à 20 % en poids par rapport au poids total de la composition, de préférence allant de 0,01 % à 10 % en poids et en particulier allant de 0,05 % à 3 % en poids.

19. Composition selon l'une quelconque des revendications 1 à 18, **caractérisée par le fait qu'**elle comprend en outre au moins un agent tensioactif additionnel choisi parmi les tensioactifs anioniques, non ioniques, amphotères et leurs mélanges.

20. Composition selon la revendication 19, **caractérisée par le fait que** le ou les agents tensioactifs additionnels sont présents à une concentration allant de 0,1% à 40% en poids, de préférence de 3% à 30% en poids, et encore plus préférentiellement de 5% à 20% en poids, par rapport au poids total de la composition.

21. Composition selon l'une quelconque des revendications 1 à 20, **caractérisée par le fait qu'**elle comprend en outre au moins un additif choisi parmi les épaississants, les parfums, les agents nacrants, les conservateurs, les filtres solaires, les silicones, les céramides, les pseudocéramides, les acides gras à chaînes linéaires ou ramifiées en C₁₆-C₄₀ tels que l'acide méthyl-18 eicosanoique, les hydroxyacides, les vitamines, les provitamines telles que le panthénol, les huiles végétales, les huiles minérales, les huiles de synthèse, les agents antipelliculaires.

22. Composition selon l'une quelconque des revendications 1 à 21, **caractérisées par le fait qu'**elle se présente sous forme de shampooing, de composition lavantes pour la peau, d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

23. Utilisation d'une composition telle que définie dans l'une quelconque des revendications précédentes pour le lavage des matières kératiniques en particulier les cheveux.

24. Utilisation d'une composition telle que définie dans l'une quelconque des revendications 1 à 22 pour améliorer le démêlage ou le lissage des cheveux , ou pour apporter du volume, de la légèreté, de la douceur, de la souplesse ou de la discipline aux cheveux.

25. Procédé de traitement des matières kératiniques, telles que les cheveux, **caractérisé en ce qu'**il consiste à appliquer sur lesdites matières une composition cosmétique selon l'une des revendications 1 à 22, puis à effectuer éventuellement un rinçage.
